# FASCICULE DE BREVET EUROPEEN

(11) **EP 2 229 145 B1**
(45) Date de publication et mention de la délivrance du brevet: **27.05.2015**
(21) Numéro de dépôt: 08872355.6
(22) Date de dépôt: 09.12.2008
(51) Int. Cl.: A61K 8/97, A61Q 19/00, A61K 36/66, A61P 17/00

(54) **UTILISATION D'UN EXTRAIT DE PETALES DE COQUELICOT POUR LA NUTRITION DE L'EPIDERME**
VERWENDUNG EINES MOHNBLÜTENEXTRAKTS ZUR HAUTPFLEGE
USE OF A POPPY PETAL EXTRACT FOR SKIN NUTRITION

(30) Priorité: 11.12.2007 FR 0708617
(43) Date de publication de la demande: 22.09.2010
(73) Titulaire: SOCIETE DE RECHERCHE COSMETIQUE, 2557 Luxembourg (LU)
(72) Inventeur: LECLERE, Jacques, F-45500 Saint-gondon (FR)
(74) Mandataire: L'Helgoualch, Jean
(86) Numéro de dépôt international: PCT/FR2008/001715
(87) Numéro de publication internationale: WO 2009/101301

(56) Documents cités:
- FR-A- 2 753 374
- FR-A- 2 871 382
- GB-A- 171 920
- IT-B- 1 243 593
- SOULIMANI R ET AL: "Behavioral and pharmaco-toxicological study of Papaver rhoeas L. in mice" MEDICINAL & AROMATIC PLANTS ABSTRACTS, RESOURCES, NEW DELHI, vol. 23, no. 5, 1 octobre 2001 (2001-10-01), XP018010286 ISSN: 0250-4367

## Description

La présente invention concerne des extraits de pétales de coquelicot dans une composition pour une utilisation à titre de médicament dermatologique nutritive.

La peau comprend plusieurs couches intégrées, allant de la couche superficielle, l'épiderme (tissu épithélial), jusqu'au couches plus profondes, le derme et l'hypoderme (tissu conjonctif), et chacune possède des propriétés spécifiques permettant à l'ensemble de réagir et de s'adapter aux conditions de son environnement.

L'épiderme, principalement composé de kératinocytes (90% des cellules épidermiques), de mélanocytes (2 à 3% des cellules épidermiques) et des cellules de Langerhans, a une épaisseur variable selon les différentes parties du corps. Etant donné qu'il constitue la couche externe de la peau, l'épiderme joue un rôle fondamental pour assurer la protection et le maintien d'une bonne trophicité. C'est pourquoi de nombreuses compositions ont été mises au point afin de le protéger et d'améliorer ses fonctions, et notamment de renforcer son élasticité et sa fermeté.

Le derme, plus épais, solide, riche en nerfs, en vaisseaux sanguins et en glandes sudoripares, se compose principalement de collagène, d'élastine et de protéoglycanes. Ces trois types de molécules sont synthétisés par les fibroblastes dermiques. Les fibres de collagène, disposées en bandes entrecroisées qui représentent 70% du poids sec du derme, assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

L'évolution des cellules dermiques du tissu conjonctif, notamment les cellules de collagène et d'élastine, s'accompagne d'une perte d'élasticité de la peau et de formation de rides et ridules, puis entraîne l'apparition de marques pigmentaires, la diminution de l'épaisseur de la peau et son affaissement. De nombreux facteurs tels que l'exposition au soleil, l'environnement, notamment la pollution et le froid, ainsi que les radicaux libres, ont pour effet d'accélérer la dégradation du collagène du derme.

La capacité de la peau à remplacer le collagène endommagé diminue non seulement avec le temps mais aussi avec des facteurs environnementaux et avec l'état de stress, et des espaces et des irrégularités apparaissent progressivement dans le réseau du collagène. A l'échelle de la peau, on observe une diminution des synthèses protéiques (collagène, élastine), une diminution de la synthèse des protéoglycanes, ainsi qu'une élévation des métalloprotéinases de type MMP3. Il en résulte d'une manière générale une diminution de la capacité des cellules du tissu conjonctif à se renouveler et à réguler les échanges d'eau et de macromolécules telles que glucides, lipides et protéines.

Les kératinocytes, et d'une manière générale les cellules eucaryotes, doivent assurer leur équilibre homéostatique avec l'environnement tissulaire. Ainsi, les cellules de l'organisme, notamment les cellules dermiques, ont besoin d'un apport équilibré de nutriments pour pouvoir fonctionner correctement. En plus des ions et des vitamines, les cellules ont besoin des macromolécules qui entrent dans la constitution cellulaire.

Si l'absorption d'aliments par la voie orale et leur assimilation par l'organisme peut apporter à l'hypoderme les nutriments qui sont nécessaires à la croissance et la régénération cellulaire, il n'en est pas de même pour le derme et, surtout, l'épiderme en raison de la plus faible vascularisation de ces couches de la peau. Or une nutrition insuffisante de la peau entraîne une perte de souplesse, d'élasticité et de tonicité. Il est donc souhaitable de pouvoir disposer de compositions topiques à effet nutritif de la peau favorisant l'équilibre physiologique de l'épiderme.

Le brevet FR 2.694.692 décrit une composition pour la nutrition de la peau associant des acides gras essentiels, des oligo-éléments et des composés vitaminés. Le brevet FR 2.857.978 décrit des compositions à base d'extraits peptidiques de spiruline présentant une activité stimulante sur les cellules du derme, utilisables pour restructurer, raffermir et hydrater la peau. Selon le brevet FR 2.753.374, des compositions associant une huile peroxygénée telle que l'huile de maïs et des extraits végétaux, permettent de renforcer les effets des extraits végétaux, notamment des hydrolysats de céréales à action nutritive. Le brevet IT 1243593 décrit une lotion fortifiante anti-rides préparée à partir d'une infusion de feuilles et de pétales séchés de rose, coquelicot, sage, romarin et menthe.

Cependant, malgré les diverses compositions disponibles, il existe toujours un besoin de pouvoir disposer de nouvelles compositions topiques alternatives, et notamment des compositions topiques à base d'extraits végétaux susceptibles d'avoir des effets favorables sur la nutrition cellulaire.

Le coquelicot (Papaver rhoeas) contient de la rhoeadine, alcaloïde de la série des tétrahydrobenzazépines, qui est supposée avoir des propriétés neuroleptiques. Cette plante a été décrite comme pouvant être utilisée dans des cas d'éréthisme cardiaque de l'adulte, comme calmant chez les adultes et les enfants, et aussi pour le traitement symptomatique de la toux.

Des compositions comprenant des extraits de coquelicot (Papaver rhoeas) en association avec des extraits de lotus bleu (Nymphaea caerulea), présentant des effets d'inhibition des contractions musculaires faciales, sont décrites dans le brevet FR 2.871.382. Ces extraits sont obtenus à partir des graines des plantes. Une composition pharmaceutique administrable par voie orale à base d'extraits de pétales de coquelicot a été proposée dans le brevet GB 171920 pour le traitement de la tuberculose. Une étude pharmaco-toxicologique chez la souris a montré que des extraits de pétales de coquelicot pouvaient avoir un effet sédatif à des doses importantes (R. Soulimari et al., "Médicinal and Aromatic Plants Abstracts", vol. 23 n° 5, oct. 2001).

Les études effectuées par la demanderesse ont montré que des extraits provenant spécifiquement des pétales de coquelicot présentaient de manière étonnante des propriétés utiles en agissant favorablement sur la nutrition cellulaire.

L'invention a pour objet l'utilisation d'un extrait de pétales de coquelicot (Papaver rhoeas) pour la préparation d'une composition dermatologique destinée à favoriser la nutrition, la stimulation de la croissance et la régénération cellulaire dermique et épidermique.

Les études effectuées, comme décrit ci-après, ont en effet mis en évidence d'intéressantes propriétés des extraits de pétales de coquelicot (Papaver rhoeas) et plus particulièrement un effet nutritif sur les cellules, procurant une activité nourrissante, un effet réparateur procurant une activité régénératrice et un effet modulateur de l'activité cellulaire utile pour stimuler la croissance des cellules. Ces effets ont été mis en évidence par l'évaluation de la vitesse de transport des macromolécules telles que glucides, lipides et protéines, au niveau des microsomes isolés et des kératinocytes humains normaux.

Cette activité permet d'évaluer l'état du métabolisme cellulaire. La communication cellulaire est une composante du métabolisme car, même si la synthèse des macromolécules est activée, une activation du flux moléculaire entre les différents organites et entre les milieux extracellulaire et intracellulaire constitue une étape importante du métabolisme cellulaire.

Les microsomes membranaires sont couramment utilisés pour l'exploration des effets de produits nouveaux sur le transport cellulaire et pour l'obtention de données préliminaires sur les interactions susceptibles de survenir entre différentes substances. Les microsomes de kératinocytes humains constituent une fraction membranaire, qui peut être obtenue par centrifugation différentielle à haute vitesse d'un homogénat de cellules.

Les résultats décrits en détail ci-après ont été obtenus in vitro en utilisant un extrait de pétales de coquelicot (Papaver rhoeas) à diverses concentrations, et ils confirment que, en application topique, un extrait de pétales de coquelicot présente un effet nutritif et régénérateur cellulaire. Ils montrent en particulier que, sur un système a cellulaire (effet direct sur les microsomes) et sur un système cellulaire (effet sur la cellule avant séparation des microsomes), la vitesse de transport des macromolécules (glucides, lipides et protéines) est augmentée de manière significative, traduisant un effet confirmé sur la nutrition cellulaire.

Il faut noter que les études effectuées sur des extraits de graines de coquelicot (Papaver rhoeas), à la différence des pétales, et plus particulièrement du mucilage, n'ont pas mis en évidence un tel effet nutritif et régénérateur cellulaire. Les graines contiennent principalement des protéines et de la rhoeadine tandis que les pétales, et en particulier le mucilage, contiennent des polysaccharides.

L'extrait de pétales de coquelicot de l'invention est de préférence un extrait aqueux, obtenu à partir de mucilage. Suivant une méthode préférée, les pétales sont extraits par l'eau en pH acide (4,6 - 6,6), ce qui permet d'obtenir les oses neutres, puis décoloration. Ainsi l'extrait est totalement aqueux. Il peut être conservé en présence d'un conservateur autorisé pour les produits biologiques (benzoate de soude et sorbate de potassium).

L'extrait de mucilage de pétales de coquelicot se présente sous la forme d'un liquide se caractérisant par :
- matières sèches 0,5 à 2%
- densité 0,980 à 1,020
- indice de réfraction (à 22°C) 1,320 à 1,350
- teneur en oses neutres 15 à 45% (moyenne 40%)
- pH (en prise directe) : 4,6 à 6,6 (moyenne 5).

Les matières sèches peuvent représenter de 0,5 à 2% comme indiqué ci-dessus, mais plus souvent 1,5 à 2%. La teneur en oses neutres est exprimée par rapport à la matière sèche.

L'extrait de l'invention décrit ci-dessus peut éventuellement être concentré et lyophilisé.

Suivant une forme avantageuse de réalisation, l'extrait de pétales de coquelicot est complété par des principes actifs ou ingrédients auxiliaires choisis pour leurs propriétés complémentaires, afin de compléter les effets nutritifs cellulaires de la composition. Ainsi il est particulièrement avantageux de le combiner avec des quantités appropriées de protéines de graines d'amarante (Amarantus caudatus) ou des globulines de pois afin d'obtenir un effet tenseur de la peau, d'huile de Calophylum afin de renforcer l'effet anti-rides, d'Imperata cylindrica, par exemple MOIST 24® de la société Sederma, afin de favoriser l'hydratation de la peau par modification de la pression osmotique, de l'huile d'Echium pour son effet anti-inflammatoire, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

Il peut être tout particulièrement avantageux d'incorporer aux compositions contenant l'extrait de pétales de coquelicot de l'invention, des actifs de type lipides, acides aminés et glucides.

Par exemple on peut ajouter des lipides tels que de l'huile d'Inca Inchi contenant des oméga 3, 6 et 9, ou de l'huile de bourrache, d'onagre, de rosier muscat, de prune ou de cerise, ou des huiles apportant des acides gras d'origine naturelle, ou encore du beurre de graines de cupuaçu (Theobroma longifolia), riche en lipides, en particulier en acide linoléique. On peut aussi incorporer dans la composition des extraits peptidiques, des hydrolysats protéiques ou des acides aminés d'origine naturelle. Les glucides que l'on peut ajouter à la composition peuvent être choisis principalement parmi le glucose, le glycogène et le tréhalose.

Les extraits de pétales de coquelicot de l'invention sont plus particulièrement destinés à des compositions pour administration par voie topique externe, c'est-à-dire une administration pour une action localisée directe, non systémique, sans passage par le système sanguin, à la différence d'une administration orale.

Les compositions selon la présente invention peuvent se présenter sous toutes les formes galéniques usuelles adaptées à une application topique externe comprenant un support physiologiquement et cosmétiquement acceptable, c'est-à-dire compatible avec la peau.

Elles peuvent comprendre entre 0,1% et 10% en poids d'extrait de pétales de coquelicot, tel que défini ci-dessus, par rapport au poids total de la composition, et de préférence entre 0,5 et 6% en poids. Le choix de la dose dans la composition peut être fait en fonction de l'utilisation envisagée.

Les compositions conformes à la présente invention peuvent être présentées sous les formes classiquement utilisées pour une application topique, c'est-à-dire sous forme de solution aqueuse ou hydroalcoolique, de gel, lotion, émulsion (en particulier crème ou lait), bâtonnet pour les lèvres, masque, pommade, sérum, patchs transdermiques, nanocapsules ou liposomes contenant des excipients et supports usuels compatibles et pharmaceutiquement acceptables. Elles peuvent aussi se présenter sous forme de lingettes imbibées d'une solution contenant l'extrait de pétales de coquelicot. Ces formes d'administration par voie topique sont préparées par,les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout.

L'extrait de pétales de coquelicot de l'invention peut être utilisé avantageusement sous une forme encapsulée dans des liposomes. Suivant une technique connue dans la fabrication des compositions cosmétiques, les liposomes sont constitués par des petites sphères creuses, de diamètre généralement inférieur à 500 nm, dont la paroi est formée d'une double couche de lipides tels que des glucolipides ou des phospholipides, c'est-à-dire de nature proche de celle de la membrane cellulaire, facilitant la pénétration dans la peau. Ils peuvent être obtenus par exemple par traitement aux ultrasons d'un mélange d'un soluté aqueux et de lipides. Les lipides (phospholipides ou glucolipides) se réorganisent dans une configuration où l'énergie de l'ensemble est minimale, donc thermodynamiquement la plus stable. Les liposomes sont utilisés dans l'industrie cosmétique pour délivrer des composés à l'intérieur des cellules lorsque le vésicule fusionne avec la membrane plasmique.

Les compositions topiques selon l'invention peuvent par exemple comprendre des excipients appropriés pour une administration topique externe, en particulier des excipients acceptables sur le plan dermatologique et cosmétologique. Ces excipients appropriés pour la formulation sont bien connus de l'homme du métier et comprennent en particulier des agents de pénétration tels que l'éthoxydiphénol, le phytantriol, l'octyl dodécanol et l'escine ; les épaississants tels que les gommes naturelles et les polymères de synthèse ; les émollients et les tensioactifs tels que l'octanoate de cétéaryle, le myristate d'isopropyle, l'isononanoate de cétéaryle, la diméthicone, la cyclométhicone, le 3-diisostéarate de poly-glycéryle, le polyisobutène hydrogéné, l'alcool cétylique, le palmitate cétylique, le phosphate cétylique ; les émulsionnants ; les conservateurs tels que le phénoxyéthanol, le parahydroxybenzoate de méthyle (méthylparaben), le parahydroxybenzoate d'éthyle (éthylparaben), le parahydroxybenzoate de propyle (propylparaben) et le Phenonip® associant du phénoxyéthanol et des parahydroxybenzoates de méthyle, éthyle, butyle et isobutyle ; les colorants ; les parfums ; etc.

D'autres ingrédients peuvent être utilisés dans les compositions : les agents hydratants tels que le propylène glycol, la glycérine, le butylène glycol et également les vitamines antioxydantes telles que la vitamine E, par exemple l'acétate de tocophérol ou le tocotriénol, la vitamine C, les polyphénols naturels. On peut également ajouter à la composition des agents conditionneurs de la peau tels que le nylon et le nitrure de bore.

Les exemples suivants illustrent l'invention plus en détail sans en limiter la portée. Dans tous les exemples de compositions qui suivent, les parties sont exprimées en poids, sauf indication contraire.

### Exemple 1

Suivant les techniques classiques, on prépare un lait démaquillant contenant un extrait de pétales de coquelicot, ayant la composition pondérale suivante.

| Phase A | |
|---|---|
| Arachidyl glucoside | 1,5 |
| Cetearyl glucoside | 1,0 |
| Alcool béhénylique | 2,0 |
| Beurre de Cupuaçu | 1,0 |
| Huile de bourrache | 1,0 |
| Triglycérides caprique / caprylique | 3,0 |
| Tocophérol | 00,5 |

| Phase B | |
|---|---|
| Eau déminéralisée | q.s.p. 100,0 |
| Glycérine | 3,0 |
| Acide phytique | 0,2 |
| Acide déhydroacétique | 0,2 |
| Benzoate de sodium | 0,2 |
| Gomme xanthane | 0,1 |

| Phase C | |
|---|---|
| Extrait de pétales de coquelicot | 3,0 |
| Eau de roses | 20,0 |

Les composants de la phase A huileuse sont mélangés à une température de 70°C, puis on y ajoute la phase B aqueuse préalablement mélangée de manière homogène à 70°C. On ajoute ensuite l'extrait de pétales de coquelicot et l'eau de roses à 40°C environ, puis éventuellement 0,05% d'essence de géranium en opérant à 37°C. Le pH est ajusté à 5,5 - 6 par addition de potasse ou d'acide citrique.

L'extrait de pétale de coquelicot utilisé ci-dessus est un extrait aqueux, obtenu par extraction par l'eau en pH acide puis décoloration, suivant une technique standard.

Le lait démaquillant ayant la composition indiquée ci-dessus peut être utilisé en application sur le visage, une à deux fois par jour.

### Exemple 2

Suivant les techniques classiques, on prépare une crème nutritive ayant la composition pondérale suivante.

| Phase A | |
|---|---|
| Lécithine hydrogénée + C₁₀-C₂₀ alcool (Biophilic H de Lucas Meyer) | 4,0 |
| Huile de bourrache | 2,0 |
| Huile d'Inca inchi | 2,0 |
| Huile de prune | 1,0 |
| Macérat huileux de souci | 0,5 |
| Beurre de Cupuaçu | 3,0 |
| Alcool béhénylique | 2,0 |
| Huile de coprah biologique | 2,0 |
| Tocophérol | 0,7 |

| Phase B | |
|---|---|
| Eau déminéralisée | q.s.p. 100,0 |
| Potasse | 0,04 |

| Phase C | |
|---|---|
| Eau déminéralisée | q.s.p. 10,0 |
| Acide déhydroacétique | 0,1 |
| Alcool benzylique | 0,5 |
| Acide phytique | 0,1 |
| Gomme xanthane | 0,1 |

| Phase D | |
|---|---|
| Glycogène | 1,0 |
| Peptides de graines d'amarante | 0,5 |
| Eau de roses | 10,0 |
| Extrait de pétales de coquelicot | 5,0 |

Les composants de la phase A huileuse sont mélangés à une température de 75°C, la phase B aqueuse préalablement mélangée de manière homogène à 75°C y est ajoutée, puis la phase C après homogénéisation à 50°C, et enfin la phase D contenant l'extrait de pétales de coquelicot à 45°C, puis éventuellement 0,02% d'essence de rose en opérant à 30°C.

L'extrait de pétales de coquelicot utilisé dans la composition ci-dessus est identique à celui de l'exemple 1.

### Exemple 3

On prépare une lotion tonique ayant la composition pondérale suivante en mélangeant successivement les composants indiqués.

| | |
|---|---|
| Eau de Roses | 30,0 |
| Eau de bleuet | 10,0 |
| Extrait de pétales de coquelicot | 5,0 |
| Extrait de fleur de nénuphar | 2,0 |
| Extrait de racine de guimauve | 1,0 |
| Sérine | 0,02 |
| Lysine | 0,02 |
| Glycocolle | 0,02 |
| Glycérine | 3,0 |
| Alcool benzylique | 0,9 |
| Acide phytique | 0,1 |

### Exemple 4

On prépare un sérum activateur de nutrition ayant la composition pondérale suivante.

| A. Poudre | |
|---|---|
| Lyophilisat d'extrait de pétales coquelicot | 0,5 |
| Mannitol | 0,5 |

| B. Solvant pour 100 g | |
|---|---|
| Eau de roses | q.s.p. 100,0 |
| Glycocolle | 0,05 |
| Tréhalose | 0,5 |
| Glycogène | 1,0 |
| Huile de bourrache | 2,0 |
| Benzoate de sodium | 0,1 |
| Sorbate de potassium | 0,1 |
| Glycérine | 3,0 |
| Acide citrique | 0,1 |

Le pH de la solution est ajusté à 5,5 environ par l'acide citrique. L'extrait de pétales de coquelicot utilisé dans la composition ci-dessus est identique à celui de l'exemple 1.

### Exemple 6

### Effets sur les microsomes isolés

L'étude consiste en une évaluation de la vitesse de transport des macromolécules (glucides, lipides et protéines) sur des microsomes isolés. L'étude a été faite en utilisant des systèmes de membranes séparés à partir de kératinocytes humains, c'est-à-dire des microsomes, et des marqueurs radioactifs ou fluorescents. Les marqueurs radioactifs [³H]-3-O-Méthyl-Glucose et [³H] choline ont été utilisés pour évaluer la vitesse de transport des glucides et des lipides, respectivement, et le marqueur fluorescent albumine-FITC pour les protéines.

### Protocole expérimental

### Transport des glucides

L'essai est conduit en triplicate après traitement direct des microsomes sur 8 lots constitués comme suit :
- lot 1 : témoin négatif ne recevant aucun produit.
- lot 2 : témoin positif (inhibition par la phloretine)
- lots 3-5 : traités par l'extrait de pétales de coquelicot de l'invention (3 concentrations : 0,1%, 0,2% et 0,5%)
- lots 6-8 : traités par la phloretine et l'extrait de l'invention (3 concentrations).

La phloretine est utilisée à la concentration de 0,25 mM.

La vitesse du transport des glucides a été évaluée comme suit :
Les microsomes obtenus à partir de kératinocytes humains en culture ont été rincés trois fois avec un tampon PBS sans glucose, puis préincubés dans 1 ml du même tampon pendant 30 minutes à 37°C. Après élimination de la solution, les microsomes sont mis dans le tampon PBS sans glucose contenant du 3-O-méthyl-glucose (MG) et du [³H]-3-O-MG sous agitation sur bain marie à 37°C.

La capture du [³H]-3-O-MG est arrêtée par ajoute de 1 ml de PBS froid contenant de la cytochalasin B. La cinétique d'incubation est réalisée entre 30 et 120 secondes.

Les microsomes sont ensuite rincés deux fois avec du PBS, puis dissous dans NaOH 1M à 4°C pendant une nuit. La radioactivité est mesurée par un compteur à scintillation.

Le traitement des microsomes par les lots définis ci-dessus est effectué en même temps que l'introduction de [³H] - 3-O-MG dans le milieu d'incubation.

Le dosage des protéines a été réalisé suivant la méthode de Bradford. L'augmentation de l'absorbance à 595 nm est proportionnelle à la concentration des protéines déterminée à l'aide d'un spectrophotomètre.

### Transport des lipides

L'essai est conduit en triplicate après traitement direct des microsomes sur 8 lots constitués comme suit :
- lot 1 : témoin négatif ne recevant aucun produit.
- lot 2 : témoin positif (inhibition par la diphenhydramine)
- lots 3-5 : traités par l'extrait de pétales de coquelicot de l'invention (3 concentrations : 0,1%, 0,2% et 0,5%)
- lots 6-8 : traités par la phloretine et l'extrait de l'invention (3 concentrations).

La diphenhydramine est utilisée à la concentration de 1 mM.

La vitesse de transport des lipides est évaluée par la même technique que ci-dessus pour les glucides, en remplaçant le tampon PBS sans glucose par du tampon Tris 25 mM et le 3-O-MG par la [³H] choline, la capture de la choline étant arrêtée par ajout d'un tampon de lyse (50 mM de Tris, 140 mM de NaCl, 1,5 mM de MgSO₄ 0,5% Igepal-Ca-630, 0,2% SDS).

Comme ci-dessus, le traitement des microsomes par les lots définis ci-dessus est effectué en même temps que l'introduction de [³H]-choline dans le milieu d'incubation.

### Transport des protéines (albumine)

L'essai est conduit en triplicate après traitement direct des microsomes sur 8 lots constitués comme suit :
- lot 1 : témoin négatif ne recevant aucun produit.
- lot 2 : témoin positif (inhibition par le nocodazole)
- lots 3-5 : traités par l'extrait de pétales de coquelicot de l'invention (3 concentrations : 0,1%, 0,2% et 0,5%)
- lots 6-8 : traités par la phloretine et l'extrait de l'invention (3 concentrations).

Le nocodazole est utilisé à la concentration de 6 mg/ml.

La vitesse de transport des protéines est évaluée par la même technique que ci-dessus pour les glucides, en remplaçant le tampon PBS sans glucose par du tampon HBSS et le 3-O-MG par l'albumine FITC, la capture de l'albumine étant arrêtée par ajout d'une solution de Ringer (125 mM de NaCl, 5,4 mM de KCl, 1,2 mM de CaCl₂, 0,8 mM de MgCl₂, 0,8 mM de Na₂PO₄, 0,2 mM de NaHPO₄, 5,5mM de glucose,10 mM HEPES, pH 7,4).

Les microsomes sont ensuite traités par du Triton X-100 (0,1% v/v dans l'acide 3-N-morpholino propanesulfonique, 20 mM, pH 7,4. La fluorescence est mesurée par un spectro-fluoromètre (excitation 430 nm ; émission 520 nm).

Comme ci-dessus, le traitement des microsomes par les lots définis ci-dessus est effectué en même temps que l'introduction de l'albumine FITC dans le milieu d'incubation.

### Résultats

### Transport des glucides

Les résultats sont regroupés dans le tableau 1 ci-dessous :

**Tableau 1**

| | capture du [³H]-3-O-MG (nmol/mg de protéine) | | | |
|---|---|---|---|---|
| | 30s | 60s | 90s | 120s |
| Témoin négatif | 13 | 19 | 23 | 27 |
| Extrait Inv. (0,1%) | 14 | 18 | 23 | 28 |
| Extrait Inv. (0,2%) | 13 | 19 | 24 | 28 |
| Extrait Inv. (0,5%) | 15 | 20 | 24 | 27 |

L'extrait de l'invention est l'extrait de pétales de coquelicot utilisé dans la composition de l'Exemple 1.

Les résultats regroupés au tableau ci-dessus, obtenus après traitement direct des microsomes de kératinocytes par l'extrait aqueux de l'invention aux concentrations de 0,1%, 0,2% et 0,5%, montrent que l'extrait n'entraîne aucune inhibition de la vitesse de transport du glucose.

La cinétique de la capture du glucose, dans les conditions physiologiques, est en effet pratiquement identique entre les microsomes témoins et les microsomes traités par l'extrait de l'invention aux trois concentrations étudiées.

Les résultats regroupés au Tableau 2 ci-dessous montrent les effets inhibiteurs connus de la phloretine et l'effet de l'extrait de l'invention.

**Tableau 2**

| | capture du [³H]-3-O-MG (nmol/mg de protéine) | | | |
|---|---|---|---|---|
| | 30s | 60s | 90s | 120s |
| Témoin négatif | 13 | 19 | 23 | 27 |
| Phloretine (0,25 mM) | 8 | 10 | 12 | 12 |
| Extrait Inv.(0,1%)+phloretine | 9 | 15 | 21 | 23 |
| Extrait Inv.(0,2%)+phloretine | 10 | 16 | 22 | 25 |
| Extrait Inv.(0,5%)+phloretine | 11 | 18 | 22 | 26 |

Comme le montrent les résultats du Tableau 2, le traitement direct des microsomes par la phloretine inhibe fortement la vitesse de transport du glucose. Par contre, le traitement par l'extrait de pétales de coquelicot de l'invention, aux trois concentrations testées, en présence de phloretine, rétablit la vitesse de transport du glucose de manière significative. Ainsi, la vitesse de transport du glucose est parfaitement rétablie par l'extrait de l'invention malgré la présence de l'inhibiteur phloretine.

### Transport des lipides

Les résultats sont regroupés dans le tableau 3 ci-dessous :

**Tableau 3**

| | capture du [³H]-choline (pmol/mg de protéine) | | | |
|---|---|---|---|---|
| | 30s | 60s | 90s | 120s |
| Témoin négatif | 165 | 210 | 272 | 312 |
| Extrait Inv. (0,1%) | 188 | 222 | 278 | 321 |
| Extrait Inv. (0,2%) | 174 | 220 | 286 | 331 |
| Extrait Inv. (0,5%) | 202 | 252 | 291 | 321 |

L'extrait de l'invention est l'extrait de pétales de coquelicot utilisé dans la composition de l'Exemple 1.

Les résultats regroupés au tableau ci-dessus, obtenus après traitement direct des microsomes de kératinocytes par l'extrait aqueux de l'invention aux concentrations de 0,1%, 0,2% et 0,5%, montrent que l'extrait n'entraîne aucune inhibition de la vitesse de transport des lipides.

La cinétique de la capture de la choline, dans les conditions physiologiques, est en effet pratiquement identique entre les microsomes témoins et les microsomes traités par l'extrait de l'invention aux trois concentrations étudiées.

Les résultats regroupés au Tableau 4 ci-dessous montrent les effets inhibiteurs connus de la diphenhydramine (DPA) et l'effet de l'extrait de l'invention.

**Tableau 4**

| | capture du [³H]-choline (pmol/mg de protéine) | | | |
|---|---|---|---|---|
| | 30s | 60s | 90s | 120s |
| Témoin négatif | 165 | 210 | 272 | 312 |
| Diphenhydramine DPA (0,25 mM) | 83 | 115 | 141 | 153 |
| Extrait Inv.(0,1%) + DPA | 112 | 132 | 163 | 195 |
| Extrait Inv.(0,2%) + DPA | 124 | 142 | 179 | 208 |
| Extrait Inv.(0,5%) + DPA | 132 | 152 | 198 | 210 |

Les résultats du Tableau 4 montrent que le traitement direct des microsomes par la diphenhydramine inhibe fortement la vitesse de transport des lipides. Par contre, le traitement par l'extrait de pétales de coquelicot de l'invention, aux trois concentrations testées, en présence de diphenhydramine, rétablit la vitesse de transport des lipides. Ainsi, la vitesse de transport des lipides est rétablie par l'extrait de l'invention malgré la présence de l'inhibiteur diphenhydramine.

### Transport des protéines

Les résultats sont regroupés dans le tableau 5 ci-dessous :

**Tableau 5**

| | capture de l'albumine FITC (µg/mg de protéine) | | | |
|---|---|---|---|---|
| | 30s | 60s | 90s | 120s |
| Témoin négatif | 0,51 | 0,64 | 0,76 | 0,96 |
| Extrait Inv. (0,1%) | 0,52 | 0,66 | 0,78 | 0,95 |
| Extrait Inv. (0,2%) | 0,56 | 0,71 | 0,83 | 0,96 |
| Extrait Inv. (0,5%) | 0,62 | 0,74 | 0,86 | 0,97 |

L'extrait de l'invention est l'extrait de pétales de coquelicot utilisé dans la composition de l'Exemple 1.

Les résultats regroupés au tableau ci-dessus, obtenus après traitement direct des microsomes de kératinocytes par l'extrait aqueux de l'invention aux concentrations de 0,1%, 0,2% et 0,5%, montrent que l'extrait n'entraîne aucune inhibition de la vitesse de transport des protéines.

La cinétique de la capture de l'albumine, dans les conditions physiologiques, est en effet pratiquement identique entre les microsomes témoins et les microsomes traités par l'extrait de l'invention aux trois concentrations étudiées.

Les résultats regroupés au Tableau 6 ci-dessous montrent les effets inhibiteurs connus du nocodazole (ND) et l'effet de l'extrait de l'invention.

**Tableau 6**

| | capture de l'albumine FITC (µg/mg de protéine) | | | |
|---|---|---|---|---|
| | 30s | 60s | 90s | 120s |
| Témoin négatif | 0,51 | 0,64 | 0,76 | 0,96 |
| Nocodazole ND (0,25 mM) | 0,31 | 0,49 | 0,54 | 0,63 |
| Extrait Inv.(0,1%) + ND | 0,22 | 0,50 | 0,53 | 0,61 |
| Extrait Inv.(0,2%) + ND | 0,39 | 0,53 | 0,58 | 0,72 |
| Extrait Inv.(0,5%) + ND | 0,43 | 0,58 | 0,64 | 0,77 |

Les résultats du Tableau 6 montrent que le traitement direct des microsomes par le nocodazole inhibe fortement la vitesse de transport des lipides. Par contre, le traitement par l'extrait de pétales de coquelicot de l'invention, aux trois concentrations testées, en présence de nocodazole, rétablit la vitesse de transport des lipides. Aucun effet n'est cependant observé à la concentration de 0,1%. Ainsi, la vitesse de transport des lipides est rétablie par l'extrait de l'invention aux concentrations de 0,2% et 0,5%, malgré la présence de l'inhibiteur nocodazole.

### Exemple 7

### Effets sur les kératinocytes humains normaux

L'étude consiste en une évaluation de la vitesse de transport des macromolécules (glucides, lipides et protéines) sur des kératinocytes humains normaux.

### Protocole expérimental

La méthode utilisée est celle des explants permettant d'obtenir à partir d'une biopsie de la peau humaine, des primo cultures de kératinocytes. Les essais ont été effectués sur des kératinocytes entre le 2^{ème} et le 4^{ème} passage afin d'assurer une reproductibilité entre les différentes expériences.

Les kératinocytes ont été répartis dans des boîtes multipuits (6 puits) à raison de 10⁵ cellules par puits dans 1 ml de milieu de culture KGM (Clonetics) supplémenté par de l'insuline et de l'hydrocortisone. Les cellules ont été incubées en présence et en l'absence de l'extrait à étudier.

### Transport des glucides

L'essai est conduit en triplicate après traitement direct des microsomes sur 8 lots constitués comme dans l'Exemple 5.

La vitesse de transport des glucides a été évaluée comme suit :

Le traitement des kératinocytes par l'extrait aqueux de pétales de coquelicot de l'invention, en présence et en l'absence de phlorétine (0,25 mM) a été effectué pendant 20 minutes à 37°C. Les microsomes membranaires ont été séparés par centrifugation différentielle.

Le même protocole de capture du [³H]-3-O-Méthyl Glucose que dans l'Exemple 5, est utilisé pour évaluer la vitesse de transport du glucose.

### Transport des lipides

L'essai est conduit en triplicate après traitement direct des microsomes sur 8 lots constitués comme dans l'Exemple 5.

La vitesse de transport des lipides a été évaluée comme suit :
Le traitement des kératinocytes par l'extrait aqueux de pétales de coquelicot de l'invention, en présence et en l'absence de diphenhydramine (1 mM) a été effectué pendant 120 minutes à 37°C. Les microsomes membranaires ont été séparés par centrifugation différentielle.

Le même protocole de capture de la [³H]-choline que dans l'Exemple 5, est utilisé pour évaluer la vitesse de transport de la choline.

### Transport des protéines (albumine)

L'essai est conduit en triplicate après traitement direct des microsomes sur 8 lots constitués comme dans l'Exemple 5.

La vitesse de transport des protéines a été évaluée comme suit :
Le traitement des kératinocytes par l'extrait aqueux de pétales de coquelicot de l'invention, en présence et en l'absence de nocodazole (1 mM) a été effectué pendant 120 minutes à 37°C. Les microsomes membranaires ont été séparés par centrifugation différentielle.

Le même protocole de capture de l'albumine FITC que dans l'Exemple 5, est utilisé pour évaluer la vitesse de transport de l'albumine.

### Résultats

### Transport des glucides

Les résultats sont regroupés au tableau 7 ci-dessous :

**Tableau 7**

| | capture du [³H]-3-O-MG (nmol/mg de protéine) | | | |
|---|---|---|---|---|
| | 30s | 60s | 90s | 120s |
| Témoin négatif | 10 | 16 | 19 | 33 |
| Extrait Inv. (0,1%) | 11 | 14 | 20 | 21 |
| Extrait Inv. (0,2%) | 12 | 17 | 21 | 22 |
| Extrait Inv. (0,5%) | 14 | 18 | 22 | 21 |

L'extrait de l'invention est l'extrait de pétales de coquelicot utilisé dans la composition de l'Exemple 1.

Les résultats regroupés au tableau ci-dessus, obtenus après traitement des kératinocytes normaux par l'extrait aqueux de l'invention aux concentrations de 0,1%, 0,2% et 0,5%, confirment que l'extrait n'entraîne aucune inhibition de la vitesse de transport du glucose.

La cinétique de la capture du glucose, dans les conditions physiologiques, est en effet pratiquement identique entre les microsomes des kératinocytes témoins et les microsomes des kératinocytes traités par l'extrait de l'invention aux trois concentrations étudiées.

Les résultats regroupés au Tableau 8 ci-dessous montrent les effets inhibiteurs connus de la phloretine et l'effet de l'extrait de l'invention.

**Tableau 8**

| | capture du [³H]-3-O-MG (nmol/mg de protéine) | | | |
|---|---|---|---|---|
| | 30s | 60s | 90s | 120s |
| Témoin négatif | 10 | 16 | 19 | 23 |
| Phloretine (0,25 mM) | 9 | 12 | 13 | 14 |
| Extrait Inv.(0,1%)+phloretine | 10 | 14 | 19 | 22 |
| Extrait Inv.(0,2%)+phloretine | 11 | 17 | 20 | 23 |
| Extrait Inv.(0,5%)+phloretine | 12 | 18 | 21 | 22 |

Comme le montrent les résultats du Tableau 8, le traitement direct des kératinocytes normaux par la phloretine préalablement à la séparation des microsomes inhibe fortement la vitesse de transport du glucose. Par contre, le traitement par l'extrait de pétales de coquelicot de l'invention, aux trois concentrations testées, en présence de phloretine, rétablit la vitesse de transport du glucose de manière significative. Ainsi, la vitesse de transport du glucose est parfaitement rétablie par l'extrait de l'invention malgré la présence de l'inhibiteur phloretine.

### Transport des lipides

Les résultats sont regroupés dans le tableau 9 ci-dessous :

**Tableau 9**

| | capture du [³H]-choline (pmol/mg de protéine) | | | |
|---|---|---|---|---|
| | 30s | 60s | 90s | 120s |
| Témoin négatif | 161 | 195 | 221 | 237 |
| Extrait Inv. (0,1%) | 173 | 189 | 219 | 240 |
| Extrait Inv. (0,2%) | 181 | 190 | 223 | 237 |
| Extrait Inv. (0,5%) | 191 | 205 | 228 | 241 |

L'extrait de l'invention est l'extrait de pétales de coquelicot utilisé dans la composition de l'Exemple 1.

Les résultats regroupés au tableau ci-dessus, obtenus après traitement des kératinocytes normaux par l'extrait aqueux de l'invention aux concentrations de 0,1%, 0,2% et 0,5%, préalablement à la séparation des microsomes, montrent que l'extrait n'entraîne aucune inhibition de la vitesse de transport des lipides.

La cinétique de la capture de la choline, dans les conditions physiologiques, est en effet pratiquement identique entre les microsomes des kératinocytes témoins et les microsomes des kératinocytes traités par l'extrait de l'invention aux trois concentrations étudiées.

Les résultats regroupés au Tableau 10 ci-dessous montrent les effets inhibiteurs connus de la diphenhydramine (DPA) et l'effet de l'extrait de l'invention.

**Tableau 10**

| | capture du [³H]-choline (pmol/mg de protéine) | | | |
|---|---|---|---|---|
| | 30s | 60s | 90s | 120s |
| Témoin négatif | 161 | 195 | 221 | 237 |
| Diphenhydramine DPA (0,25 mM) | 113 | 134 | 165 | 175 |
| Extrait Inv.(0,1%) + DPA | 131 | 149 | 180 | 193 |
| Extrait Inv.(0,2%) + DPA | 140 | 153 | 184 | 200 |
| Extrait Inv.(0,5%) + DPA | 149 | 160 | 193 | 209 |

Les résultats du Tableau 10 montrent que le traitement des kératinocytes normaux par la diphenhydramine, préalablement à la séparation des microsomes, inhibe modérément la vitesse de transport des lipides. Par contre, le traitement par l'extrait de pétales de coquelicot de l'invention, aux trois concentrations testées, en présence de diphenhydramine, rétablit la vitesse de transport des lipides. Ainsi, la vitesse de transport des lipides est rétablie par l'extrait de l'invention malgré la présence de l'inhibiteur diphenhydramine.

### Transport des protéines

Les résultats sont regroupés au tableau 11 ci-dessous :

**Tableau 11**

| | capture de l'albumine FITC (µg/mg de protéine) | | | |
|---|---|---|---|---|
| | 30s | 60s | 90s | 120s |
| Témoin négatif | 0,44 | 0,58 | 0,64 | 0,82 |
| Extrait Inv. (0,1%) | 0,45 | 0,61 | 0,69 | 0,84 |
| Extrait Inv. (0,2%) | 0,49 | 0,66 | 0,74 | 0,88 |
| Extrait Inv. (0,5%) | 00,52 | 0,65 | 0,76 | 0,91 |

L'extrait de l'invention est l'extrait de pétales de coquelicot utilisé dans la composition de l'Exemple 1.

Les résultats regroupés au tableau ci-dessus, obtenus après traitement des kératinocytes normaux, préalablement à la séparation des microsomes, par l'extrait aqueux de l'invention aux concentrations étudiées, montrent que l'extrait n'entraîne pratiquement aucune inhibition de la vitesse de transport des protéines.

La cinétique de la capture de l'albumine, dans les conditions physiologiques est identique à la concentration de 0,1%, et elle n'est qu'en faible augmentation entre les microsomes des kératinocytes témoins et les microsomes des kératinocytes traités par l'extrait de l'invention aux concentrations de 0,2% et 0,5%.

Les résultats regroupés au Tableau 12 ci-dessous montrent les effets inhibiteurs connus du nocodazole (ND) et l'effet de l'extrait de l'invention.

**Tableau 12**

| | capture de l'albumine FITC (µg/mg de protéine) | | | |
|---|---|---|---|---|
| | 30s | 60s | 90s | 120s |
| Témoin négatif | 0,44 | 0,58 | 0,64 | 0,82 |
| Nocodazole ND (0,25 mM) | 0,19 | 0,42 | 0,46 | 0,57 |
| Extrait Inv.(0,1%) + ND | 0,31 | 0,45 | 0,47 | 0,58 |
| Extrait Inv.(0,2%) + ND | 0,32 | 0,49 | 0,58 | 0,71 |
| Extrait Inv.(0,5%) + ND | 0,38 | 0,51 | 0,59 | 0,73 |

Les résultats du Tableau 12 montrent que le traitement des kératinocytes normaux par le nocodazole, préalablement à la séparation des microsomes, inhibe la vitesse de transport des protéines.

Par contre, le traitement par l'extrait de pétales de coquelicot de l'invention, aux trois concentrations testées, en présence de nocodazole, rétablit la vitesse de transport des lipides aux concentrations de 0,2% et 0,5%, malgré la présence de l'inhibiteur nocodazole. Aucun effet n'est cependant observé à la concentration de 0,1%.

On constate donc que le traitement direct des microsomes, de même que le traitement de kératinocytes normaux préalablement à la spéciation des microsomes, n'induit aucune modification de la vitesse de transport des macromolécules dans des conditions physiologiques. De plus, il induit une augmentation nette de la vitesse de transport dans des conditions d'inhibition provoquées par des inhibiteurs tels que la phloretine, la diphenhydramine et le nocodazole. Cet effet est particulièrement important en ce qui concerne le transport des glucides.

Ces résultats mettent en évidence l'effet nutritif exercé par l'extrait de pétales de coquelicot de l'invention.

## Revendications

1. Composition dermatologique pour application topique, comprenant une quantité efficace d'un extrait de pétales de coquelicot (Papaver rhoeas) pour une utilisation à titre de médicament dermatologique pour favoriser la nutrition, la stimulation de la croissance et la régénération cellulaire dermique et épidermique.

2. Composition selon la revendication 1, **caractérisée en ce qu'**elle comprend un extrait de mucilage de pétales de coquelicot.

3. Composition selon la revendication 1 ou 2, **caractérisée en ce que** la concentration en extrait de pétales de coquelicot est comprise entre 0,1% et 10% par rapport au poids total de la composition.

4. Composition selon l'une quelconque des revendications 1 à 3, **caractérisée en ce que** l'extrait de coquelicot (Papaver rhoeas) se présente sous la forme d'un liquide **se caractérisant par** :
- matières sèches 0,5 à 2%
- densité 0,980 à 1,020
- indice de réfraction (à 22°C) 1,320 à 1,350
- teneur en oses neutres 15 à 45% (moyenne 40%)
- pH (en prise directe) : 4,6 à 6,6 (moyenne 5).

5. Composition selon l'une quelconque des revendications 1 à 4, **caractérisée en ce qu'**elle comprend en outre des lipides tels que de l'huile d'Inca Inchi de bourrache, d'onagre, de rosier muscat, de prune ou de cerise, ou du beurre de graines de cupuaçu (Theobroma longifolia), des extraits peptidiques, des hydrolysats protéiques ou des acides aminés d'origine naturelle, ou des glucides choisis parmi le glucose, le glycogène et le tréhalose.

6. Composition selon l'une quelconque des revendications 1 à 5, **caractérisée en ce qu'**elle comprend en outre des protéines de graines d'amarante (Amarantus caudatus) ou des globulines de pois, de l'huile de Calophylum, de l'Imperata cylindrica, de l'huile d'Echium, un palmitoyl pentapeptide-3 ou des dérivés tels que le palmitoyl GHK et le palmitoyl GQPR ou le palmitoyl VGVAPG associé à un céramide-2, ainsi qu'un céramide.

## Patentansprüche

1. Dematologische Zusammensetzung zur topischen Anwendung, die eine wirksame Menge eines Extrakts aus den Blütenblätter des Mohns (Papaver rhoeas) umfasst, für eine Verwendung als dermatologisches Arzneimittel, um die Ernährung, die Stimulierung des Wachstums und die Zellregeneration in der Dermis und Epidermis zu fördern.

2. Zusammensetzung nach Anspruch 1, **dadurch gekennzeichnet, dass** sie einen Schleimstoffextrakt aus den Blütenblättern des Mohns umfasst.

3. Zusammensetzung nach Anspruch 1 oder 2, **dadurch gekennzeichnet, dass** die Konzentration des Extrakts aus Blütenblättern des Mohns zwischen 0,1 % und 10 % inklusive im Verhältnis zum Gesamtgewicht der Zusammensetzung ist.

4. Zusammensetzung nach einem der Ansprüche 1 bis 3, **dadurch gekennzeichnet, dass** der Mohnextrakt (Papaver rhoeas) die Form einer Flüssigkeit hat, die **gekennzeichnet ist durch**:
- Trockenmassen 0,5 bis 2 %
- Dichte 0,980 bis 1,020
- Refraktionsindex (bei 22 °C) 1,320 bis 1,350
- Gehalt an neuralen Osen 15 bis 45 % (Durchschnitt 40 %)
- pH (direkte Messungen) 4,6 bis 6,6 (Durchschnitt 5)

5. Zusammensetzung nach einem der Ansprüche 1 bis 4, **dadurch gekennzeichnet, dass** sie ferner Lipide wie Inca Inchi-, Borretsch-, Nachtkerzen-, Weinrosen-, Pflaumen- oder Kirschöl, oder Butter aus Cupuaçu-Saat (Theobroma longifolia), Peptidextrakte, Proteinhydrolysate oder natürliche Aminosäuren oder Kohlenhydrate, ausgewählt aus der Glucose, dem Glycogen und der Trehalose, umfasst.

6. Zusammensetzung nach einem der Ansprüche 1 bis 5, **dadurch gekennzeichnet, dass** sie ferner Proteine aus Amaranthsamen (Amarantus caudatus) oder Erbsenglobuline, Calophyllum-, Imperata cylindrica-Öl, Echiumöl, ein Palmitoylpentapeptid-3 oder Derivate wie das Palmitoyl GHK und das Palmitoyl GQPR oder das Palmitoyl VGVAPG in Kombination mit einem Ceramid-2 sowie ein Ceramid umfasst.

## Claims

1. A dermatological composition for topical application comprising an efficient amount of poppy petal extract (*Papaver rhoeas*) for use as dermatological medication to promote the nutrition, stimulated growth and regeneration of dermal and epidermal cells.

2. The composition according to claim 1, **characterized in that** it comprises a mucilage extract of poppy petals.

3. The composition according to claim 1 or 2, **characterized in that** the concentration of poppy petal extract is between 0.1 % and 10 % relative to the total weight of the composition.

4. The composition according to any of claims 1 to 3, **characterized in that** the poppy extract (*Papaver rhoas*) is in the form of a liquid **characterized by**:
- dry matter: 0.5 to 2 %
- density: 0.980 to 1.020
- refractive index (at 22°C) 1.320 to 1.350
- neutral ose content 15 to 45 % (mean 40 %)
- pH (direct administration) 4.6 to 6.6 (mean 5)

5. The composition according to any of claims 1 to 4, **characterized in that** it further comprises lipids such as oil of Inca Inchi, of borage, evening primrose, rose hip, plum, cherry, or Cupuacu seed butter (*Theobroma longifolia),* peptide extracts, protein hydrolysates or amino acids of natural origin, or carbohydrates selected from among glucose, glycogen and trehalose.

6. The composition according to any of claims 1 to 5, **characterized in that** it further comprises proteins of amaranth seeds (*Amarantus caudatus*) or pea globulins, Calophylum oil, *Imperata cylindrica* oil, Echium oil, a palmitoyl pentapeptide-3 or derivatives such as palmitoyl- GHK and palmitoyl-GQPR or palmitoyl-VGVAPG associated with a ceramide-2, and a ceramide.
